# NEW EUROPEAN PATENT SPECIFICATION

(11) **EP 2 268 830 B2**
(45) Date of publication and mention of the opposition decision: **15.06.2016**
(45) Mention of the grant of the patent: 16.05.2012
(21) Application number: 09721862.2
(22) Date of filing: 12.03.2009
(51) Int. Cl.: C12Q 1/04, G01N 33/50, G01N 33/569, A61M 1/28, B01D 61/14

(54) **PERITONEAL DIALYSIS SOLUTION TEST METHOD**
TESTVERFAHREN FÜR PERITONEALDIALYSELÖSUNG
PROCÉDÉ DE TEST DE SOLUTIONS DE DIALYSE PÉRITONÉALE

(30) Priority: 20.03.2008 US 52446
(43) Date of publication of application: 05.01.2011
(73) Proprietor: Baxter International Inc., Deerfield, IL 60015 (US); Baxter Healthcare SA, 8152 Glattpark (Opfikon) (CH)
(72) Inventor: WANG, Run, Gurnee IL 60031 (US); HOLMES, Cliff, Glenview IL 60025 (US); SKOUFOS, Line, Mount Prospect IL 60056 (US); LAMELA, John, Burlington WI 53105 (US)
(74) Representative: Potter Clarkson LLP
(86) International application number: PCT/US2009/036940
(87) International publication number: WO 2009/117304

(56) References cited:
- WO-A1-2005/093087
- WO-A1-2007/076411
- WO-A1-2008/009292
- US-A- 5 731 164
- HOFFMANN S ET AL: "International validation of novel pyrogen tests based on human monocytoid cells" JOURNAL OF IMMUNOLOGICAL METHODS, ELSEVIER SCIENCE PUBLISHERS B.V.,AMSTERDAM, NL, vol. 298, no. 1-2, 1 March 2005 (2005-03-01), pages 161-173, XP004853246 ISSN: 0022-1759

## Description

### BACKGROUND

The present disclosure relates generally to a method of testing a dialysis solution. More particularly, the present disclosure relates to a method of testing a peritoneal dialysis solution and components thereof.

Parenteral pharmaceutical products are required to be free of contaminating substances, such as those that might cause peritonitis. Peritonitis, or inflammation of the peritoneum, is a major complication of peritoneal dialysis. Peritonitis may be caused by intraperitoneal bacterial infections. Alternatively, peritonitis caused by a chemical or a foreign body irritant is known as aseptic or sterile peritonitis. Sterile peritonitis is accompanied with development of a cloudy dialysate. Despite existing testing of peritoneal dialysis solutions, outbreaks of aseptic peritonitis still occur.

WO 2007/076411 discloses a monocyte activation test for detecting non-endotoxin pyrogens in medical products.

WO 2008/009292 discloses an in-vitro method for detecting inflammatory contaminants in a sample.

US-5,731,164 discloses a method for checking the rate of removal of pyrogenic substances such as viruses from organic material.

### SUMMARY

In one aspect, the present invention provides a method of testing a glucose polymer or a glucose polymer derivative according to claim 1.

The present disclosure relates to methods of testing peritoneal dialysis solution components. The methods in an embodiment use a proinflammatory response such as a cytokine detection test in combination with a filtering step to test for the presence of contaminants in a dialysis solution component.

The proinflammatory response is interleukin-6.

In an embodiment, the sample is a first sample and the method further includes providing a second sample of the glucose polymer or glucose polymer derivative, adding the first sample to a third assay, adding a reagent to the third assay, and measuring the proinflammatory response of the third assay.

The reagent includes peripheral blood mononuclear cells or monocytic cell line cells.

According to the present invention, the specified molecular weight cutoff is greater than or equal to about 30 kDa and less than or equal to about 100 kDa. The specified molecular weight cutoff may be greater than or equal to about 50 kDa and less than or equal to about 100 kDa.

According to the present invention, if the proinflammatory response of the second assay, as expressed in pg/mL, is greater than or equal to 50% of the proinflammatory response of the first assay, as expressed in pg/mL, the glucose polymer or glucose polymer derivative is acceptable for use. If the proinflammatory response of the second essay, as expressed in pg/mL, is less than 50% of the proinflammatory response of the first assay, as expressed in pg/mL, the glucose polymer or glucose polymer derivative is rejected for use.

In an embodiment, the glucose polymer or glucose polymer derivative is icodextrin.

In another aspect, the present invention provides a method of testing a glucose polymer or a glucose polymer derivative according to claim 2.

In an embodiment, if the proinflammatory response induced by the filtrate portion, as expressed in pg/mL, is greater than or equal to 50% of the proinflammatory response induced by the retentate portion, as expressed in pg/mL, the glucose polymer or glucose polymer derivative is acceptable for use. If the proinflammatory response induced by the filtrate portion, as expressed in pg/mL, is less than 50% of the proinflammatory response induced by the retentate portion, as expressed in pg/mL, the glucose polymer or glucose polymer derivative is rejected for use.

The present invention also provides a method of testing a peritoneal dialysis solution according to claim 4 dialysis solutions.

Another advantage of the present disclosure is to provide improved methods for manufacturing and using peritoneal dialysis solutions that employ a detection protocol to determine the presence of contaminants in the peritoneal dialysis solution.

Yet another advantage of the present disclosure is to provide improved testing procedures that can be employed to prevent peritonitis in patients that receive peritoneal dialysis therapy.

Yet still another advantage of the present disclosure is to provide an improved icodextrin composition that utilizes a detection procedure in the manufacture thereof to determine the presence of peptidoglycan.

Additional features and advantages of the present disclosure are described in, and will be apparent from, the following Detailed Description and the figures.

### BRIEF DESCRIPTION OF THE FIGURES

FIG. 1 is a chart illustrating the interleukin-6 response for Examples 1 and 2.
FIG. 2 is a chart illustrating the interleukin-6 response for Examples 3, 4, and 5.
FIG. 3 is a chart illustrating the interleukin-1a response for Examples 3, 4, and 5.
FIG. 4 is a chart illustrating the interleukin-1b response for Examples 3, 4, and 5.
FIG. 5 is a chart illustrating the tumor necrosis factor-α response for Examples 3,4, and 5.
FIG. 6 is a chart illustrating the interleukin-10 response for Examples 3, 4, and 5.
FIG. 7 is a chart illustrating the macrophage inflammatory proteins 1a response for Examples 3, 4, and 5.
FIG. 8 is a chart illustrating the macrophage inflammatory proteins 1 b response for Examples 3, 4, and 5.
FIG. 9 is a chart illustrating the granulocyte colony-stimulating factor response for Examples 3, 4, and 5.
FIG. 10 is a chart illustrating the granulocyte-macrophage colony-stimulating factor response for Examples 3, 4, and 5.
FIG. 11 is a chart illustrating the interleukin-6 response for Examples 6 and 7.
FIG. 12 is a chart illustrating the interleukin-6 response for Examples 8 and 9.
FIG. 13 is a chart illustrating the interleukin-6 response for Examples 10 and 11.
FIG. 14 is a chart illustrating the interleukin-6 response for Examples 12 and 13.

### DETAILED DESCRIPTION

The present disclosure relates to methods of using a proinflammatory response test in combination with a filtering step to test for the presence of contaminants in dialysis solution components. The test may be performed on any desired material, but is typically performed on a dialysis fluid or the components thereof. Dialysis solutions are used in various forms of dialysis to remove waste products from a patient. Dialysis solutions can be specifically formulated and suitable for peritoneal dialysis, hemodialysis or any other dialysis therapies. The dialysis solutions can include one or more suitable dialysis components (e.g. ingredients or constituents of a dialysis solution) such as osmotic agents, buffers, electrolytes or combinations thereof. The methods disclosed herein may be used with any type of dialysis solution, and is especially useful for peritoneal dialysis solutions that contain a glucose polymer, such as icodextrin, a glucose polymer derivative, and the like.

Various compounds that are major components of a bacterial cell wall can contaminate dialysis solution products and go undetected with conventional methods. In this regard, testing for bacterial components may be used to prevent sterile peritonitis in patients that use the peritoneal dialysis solutions. A proinflammatory response can be determined using markers such as cytokines. Cytokine secretion is a common cellular response to many substances including bacterial and non-bacterial substances. Many known bacterial components such as lipopolysaccharide (LPS), and peptidoglycan (PG), stimulate cytokine production in human cells. Cytokines commonly associated with responses to bacterial components include interleukin-1a (IL-1a), interleukin-1b (IL-1b), interleukin-6 (IL-6), interleukin-10 (IL-10), tumor necrosis factor-α (TNF α), macrophage inflammatory proteins 1a and 1b (MIP-1a, MIP-1b), granulocyte colony-stimulating factor (G-CSF), and granulocyte-macrophage colony-stimulating factor (GM-CSF). Cytokine markers and the appropriate selection thereof are known to those skilled in the art.

Tests have previously been developed to measure the cytokine response of a material to detect the presence of bacterial components. It has been found that by first filtering a material to separate it into components based on molecular weight, and then measuring the proinflammatory response induced by the components, a more accurate result may be obtained. In particular, the test provides a better indication as to whether the material contains contaminants that are likely to cause aseptic peritonitis in a patient.

Generally, the test is performed on a sample of the dialysis solution component, such as a glucose polymer or a glucose polymer derivative, or solution thereof. The sample may be first assayed to determine if it initiates a proinflammatory response. If no significant proinflammatory response is indicated, the dialysis solution component may be considered safe for use in a patient. If a significant proinflammatory response is indicated, further testing may be done to determine if the dialysis solution component is likely to induce sterile peritonitis. A second sample of the dialysis solution component is filtered at a specified molecular weight cutoff to obtain a retentate portion and a filtrate portion. The proinflammatory response of each of the retentate portion and the filtrate portion is measured to obtain a proinflammatory test result for each portion. The proinflammatory response of the retentate portion is then compared to the proinflammatory response of the filtrate portion to determine if the dialysis solution is suitable for use.

The proinflammatory response may be measured using any suitable test. The proinflammatory response induced by a material may be measured using an assay system. In one type of test, a reagent that produces a cytokine response is added to a sample of the dialysis solution in an assay. The reagent includes peripheral blood mononuclear cells (PBMCs) or monocytic cell line cells. One particular type of suitable test includes a test kit with an assay. The material to be tested and PBMCs are added to the assay. PBMCs are preferably isolated from fresh human blood of healthy donors. The assays are then incubated with the cells overnight. The culture media is collected and the secreted cytokine of the desired type is quantified using Enzyme-Linked Immuno Sorbent Assay (ELISA) techniques. The use of such tests is well known in the art.

A test to measure the interleukin-6 response may be performed on the sample. Test kits are available commercially from numerous vendors such as R&D Systems, Inc., Minneapolis, MN (USA).

To perform the filtration step, any suitable filtration method may be used. A suitable device for performing the filtration is the Amicon Ultra-4 Centrifugal Filter Unit (available from Millipore™). The filter unit may include a molecular weight cutoff designated as the nominal molecular weight limit (NMWL). The molecular weight cutoff indicates the ability of the device to retain molecules above the specified molecular weight. For example, a filter with a molecular weight cut off of 5 KDa allows molecular species smaller than 5K Da to pass through the membrane and reside in the filtrate after centrifugation of the device. If the solution contains molecules close to the molecular weight cutoff, these molecules may partially pass through the filter. The samples to be tested are added to a filter unit and centrifuged to obtain a filtrate and a retentate sample. The retentate is retained in the filter media and the filtrate is collected in a centrifuge tube.

The solution may be subjected to a filtration step described above at molecular weight cutoffs of about 10 kDa, 15 kDa, 20 kDa, 25 kDa, 30 kDa, 40 kDa, 50 kDa, 60 kDa, 70 kDa, 80 kDa, 90 kDa, 100 kDa, and other appropriate molecular weight cutoffs. The molecular weight cutoff of the filter may be selected based on the size of possible contaminants, or based on other factors. The filtrate and retentate are tested for a proinflammatory response at each level. The results of the tests are compared to determine whether the dialysis solution component is suitable for use. Various standards may be used to make the determination. It has been found that if the test results at a certain molecular weight cutoff are the same for the filtrate and retentate (indicating that the contaminant is not preferentially retained by the filter), the dialysis solution component will not cause aseptic peritonitis in a patient. If the proinflammatory response for the retentate at a certain molecular weight cutoff is sufficiently greater than the proinflammatory response for the filtrate (indicating that the contaminant is larger in size than the molecular weight cutoff), the dialysis solution component may cause aseptic peritonitis in a patient and thus must be rejected. Alternatively, multiple wash or rinsing steps with molecular weight cutoff filters can be used to determine if species with large molecular weights are present in the retentate. If test of such retentate produces an elevated IL-6 response, the dialysis solution is rejected.

It is believed that contaminants in the range of 30 kDa to 100 kDa are particularly likely to cause aseptic peritonitis, while contaminants that are not preferentially retained by the filter are not likely to cause aseptic peritonitis. For example, peptidoglycan has been shown to cause aseptic peritonitis. The methods disclosed herein are capable of detecting peptidoglycan in a dialysis solution or component thereof to prevent the release of the contaminated solution.

The cytokine response is typically expressed in pg/mL. In one embodiment, the following test criteria may be used to determine the suitability of a material. If the proinflammatory response of the material is negative (less than about 100-200 pg/mL, or about the same as a negative control), the material is suitable for use. If the retentate portion exhibits a positive proinflammatory response and the filtrate portion exhibits a negative proinflammatory response, the material is rejected. If both the filtrate and retentate portions exhibit positive proinflammatory responses and the proinflammatory response of the filtrate is greater than the proinflammatory response of the retentate, the material is acceptable for use. If both the filtrate and retentate portions exhibit positive proinflammatory responses and the proinflammatory response of the filtrate is less than the proinflammatory response of the retentate, than the ratio of the filtrate proinflammatory response to the retentate proinflammatory response is examined. If the proinflammatory response of the filtrate portion is greater than or equal to 50% of the proinflammatory response of the retentate portion, the dialysis solution component is considered acceptable for use. If the proinflammatory response of the filtrate portion is less than 50% of the proinflammatory response of the retentate portion, the dialysis solution component may cause aseptic peritonitis in a user and thus must be rejected and discarded. Other criteria may be used depending on the likely source of contamination.

The criteria for determining if a material is suitable may change according to specific experimental parameters. This is because relative concentrations of molecules in the filtrate and retentate depend on the specific type of molecular weight cut off filters such as size and volume, spinning time and centrifugal force applied. The dose dependent response from a contaminant may exhibit a sigmoid function; therefore, one may have to use diluted sample if the response has reached the saturated point.

In an embodiment, the present disclosure provides methods for manufacturing a peritoneal dialysis solution. The method can include any suitable number and type of processing stages. For example, the process includes providing a glucose polymer, such as icodextrin; filtering a sample of the glucose polymer to obtain a retentate portion and a filtrate portion; measuring the proinflammatory response of each of the retentate portion and the filtrate portion; comparing the proinflammatory response of the retentate portion to the proinflammatory response of the filtrate portion; and using the glucose polymer to make peritoneal dialysis solution if it is determined that the IL-6 response is in accordance with predetermined criteria. If it is determined that the IL-6 response is not in accordance with predetermined criteria, the glucose polymer can be further processed to remove the contaminant or to achieve a sufficiently low level of the contaminant. The glucose polymer can be further processed in any suitable manner. In an embodiment, the glucose polymer can be processed with any suitable number and type of separation devices, such as affinity columns with resins that specifically bind peptidoglycan and/or the like.

The method of manufacturing a dialysis solution in accordance with the present disclosure can also be used in conjunction with other suitable dialysis raw material or dialysis solution testing procedures. Illustrative examples of suitable procedures can be found in U.S. Patent No. 7,118,857, entitled METHODS AND COMPOSITIONS FOR DETECTION OF MICROBIAL CONTAMINANTS IN PERITONEAL DIALYSIS SOLUTIONS, issued on October 10, 2006. For example, such testing procedures can be generally used to test dialysis raw materials or dialysis solutions.

The dialysis solutions can be specifically formulated and suitable for peritoneal dialysis, hemodialysis or any other dialysis therapies. The dialysis solutions can be used, for example, as a single dialysis solution in a single container or as a dialysis part of a separately housed or multi-chambered container. The dialysis solutions can be sterilized using any suitable sterilizing technique such as, for example, autoclave, steam, ultraviolet, high pressure, filtration or combination thereof.

Ready-to-use formulations of dialysis solutions can be prepared in a number of suitable ways. For example, the first and second dialysis parts can be separately stored from each other, such as in separate and hydraulically connected chambers of a multichamber container, until mixed together to form a mixed solution. In this regard, the ready-to-use formulation can be prepared within the container by mixing its separate dialysis parts within one chamber of the container. This can effectively eliminate the need to manually inject all or at least a portion of the dialysis parts into the container to form the mixed solution, thus ensuring that the ready-to-use formulation can be readily prepared under sterile conditions.

Further, the container can be configured such that one of the dialysis parts can be placed in direct fluid communication with the patient prior to mixing while the other dialysis part cannot be placed in direct fluid communication with the patient prior to mixing. This can provide an added level of safety with respect to the preparation and administration of the ready-to-use formulation of the present disclosure as the single solution that cannot be placed in direct fluid communication with the patient physically cannot be fed to the patient unless it is first mixed with the other component. In this regard, if, by chance, the single solution that physically cannot be placed in direct fluid communication with the patient were to have an undesirable concentration of constituents, such as potassium, sodium or the like, this configuration would necessarily ensure that the undesirable level of constituents is not fed or administered to the patient.

It should be appreciated that the separate dialysis parts of a multi-part dialysis solution can be housed or contained in any suitable manner such that the individual dialysis parts can be effectively prepared and administered. A variety of containers can be used to house the two parts, such as separate containers (e.g., flasks or bags) that are connected by a suitable fluid communication mechanism. The two or more separate dialysis parts can be separately sterilized and stored.

The dialysis solutions can comprise one or more suitable dialysis components (e.g. ingredients or constituents of a dialysis solution) such as osmotic agents, buffers, electrolytes or combination thereof. A variety of different and suitable acidic and/or basic agents can also be utilized to adjust the pH of the osmotic, buffer and/or electrolyte solutions or concentrates. For example, a variety of inorganic acids and bases can be utilized including hydrochloric acid, sulfuric acid, nitric acid, hydrogen bromide, hydrogen iodide, sodium hydroxide, the like or combination thereof.

Examples of osmotic agents include glucose, fructose, glucose polymers (e.g. maltodextrin, icodextrin, cyclodextrins, trehalose), glucose polymer derivatives (e.g. modified starch, hydroxyethyl starch), polyols, amino acids, peptides, proteins, amino sugars, N-acetyl glucosamine (NAG), glycerol and/or the like and combinations thereof. Examples of the buffers include bicarbonate, lactic acid/lactate, pyruvic acid/pyruvate, acetic acid/acetate, citric acid/citrate, amino acids, peptides, an intermediate of the KREBS cycle and/or the like and combinations thereof.

Examples of electrolytes include calcium, magnesium, sodium, potassium, chloride and/or the like and combinations thereof. For example, the dialysis solutions can comprise one or more electrolytes in the following ranges from: about 100 to about 140 mEq/L of Na+, about 70 to about 130 mEq/L of Cl-, 0.1 to about 4.0 mEq/L of Ca2+, 0.1 to about 4.0 mEq/L of Mg2+ and/or 0.1 to about 4.0 mEq/L of K+.

The dialysis solutions can preferably contain a dialysis component such as an osmotic agent to maintain the osmotic pressure of the solution greater than the physiological osmotic pressure (e.g. greater than about 285 mOsmol/kg). For example, glucose is the most commonly used osmotic agent because it provides rapid ultrafiltration rates. Other suitable types of osmotic agents such as amino acids can be used in addition to or as a substitute for glucose.

Another family of compounds capable of serving as osmotic agents in peritoneal dialysis solutions is that of glucose polymers or their derivatives, such as icodextrin, maltodextrins, hydroxyethyl starch, and the like. While these compounds are suitable for use as osmotic agents, they can be sensitive to low and high pH, especially during sterilization and long-term storage. Glucose polymers, such as icodextrin, can be used in addition to or in place of glucose in peritoneal dialysis solutions. In general, icodextrin is a polymer of glucose derived from the hydrolysis of corn starch. It has a molecular weight of 12-20,000 Daltons. The majority of glucose molecules in icodextrin are linearly linked with α (1-4) glucosidic bonds (>90%) while a small fraction (<10%) is linked by α (1-6) bonds.

The dialysis solutions or components can also comprise buffering agents such as bicarbonates and acids. The bicarbonates can comprise an alkaline solution such that the bicarbonate can remain stable without the use of a gas barrier overpouch or the like. The individual bicarbonate solution can have a pH that ranges above about 8.6, preferably about 9. The pH of the bicarbonate solution part can be adjusted with any suitable type of ingredient, such as sodium hydroxide and/or the like. Illustrative examples of the bicarbonate solution of the present disclosure can be found in U.S. Patent No. 6,309,673, entitled BICARBONATE-BASED SOLUTION IN TWO PARTS FOR PERITONEAL DIALYSIS OR SUBSTITUTION IN CONTINUOUS RENAL REPLACEMENT THERAPY, issued on October 30, 2001.

The acids can comprise one or more physiological acceptable acids, such as lactic acid, pyruvic acid, acetic acid, citric acid, hydrochloric acid and the like. The acids can be in an individual solution having a pH that ranges from about 5 or less, about 4 or less, about 3 or less, about 2 or less, about 1 or less, and any other suitable acidic pH. The use of an organic acid, such as lactic acid, alone or in combination with another suitable acid, such as a suitable inorganic acid including hydrochloric acid, another suitable organic acid (e.g. lactic acid/lactate, pyruvic acid/pyruvate, acetic acid/acetate, citric acid/citrate) and the like in the acid solution can make the solution more physiologically tolerable.

As discussed previously, the dialysis solutions of the present disclosure can be used in a variety of suitable applications. For example, the dialysis solutions can be used during peritoneal dialysis, such as automated peritoneal dialysis, continuous ambulatory peritoneal dialysis, continuous flow peritoneal dialysis and the like. It should be appreciated that the present disclosure can be used in a variety of different and suitable dialysis therapies to treat kidney failure.

Although the present disclosure, in an embodiment, can be utilized in methods providing a dialysis therapy for patients having chronic kidney failure or disease, it should be appreciated that the present disclosure can be used for acute dialysis needs, for example, in an emergency room setting. Lastly, as one of skill in the art appreciates, the intermittent forms of therapy (e.g., hemofiltration, hemodialysis, peritoneal dialysis and hemodiafiltration) may be used in the in center, self/limited care as well as the home settings.

### EXPERIMENTAL EXAMPLES

By way of example and not limitation, the following examples are illustrative of various embodiments of the present disclosure and further illustrate experimental testing conducted with peritoneal dialysis solutions.

### Experimental Procedure

To determine if a material would induce a cytokine response, a standard interleukin-6 (IL-6) response was performed. For a typical experiment, a total of approximately 75 mL whole blood was collected per donor. Peripheral blood mononuclear cells (PBMCs) were isolated, and the cell concentration determined.

The PBMC cell number was determined using a cell counter and adjusted to a concentration of approximately 8x10⁶ cells/mL. An aliquot of 100µL of cell suspension was added to each well and a 100µL solution made of control or test articles were added. A total of four replicates (four wells per sample or control solution) were performed in each experiment with four blood donors. The 96-well plates prepared with cells and test and control solutions were incubated in a CO₂ incubator at 5% CO₂ overnight.

After the incubation period the plates were centrifuged for fifteen minutes at 430xg. Supernatant was collected. The concentration of IL-6 in the supernatant was determined using the QuantiGlo Chemiluminescent ELISA kit. A SpectraMax M2 plate reader was set for luminescence reading.

To perform the filtration step, Amicon Ultra-4 filtration devices were used. Amicon Ultra-4 filtration devices (from Millipore™) are designed for concentration of biological samples and purification of macromolecular components. The Amicon Ultra-4 devices include membranes made of low-protein binding regenerated cellulose and characterized by a Nominal Molecular Weight Limit (NMWL). The rated NMWL indicates the ability of the device to retain molecules above the specified molecular weight. The Ultra-4 filtration devices are designed to enable a single spin recovery of the concentrated retentate (collected in the filter unit) and filtrate (collected in the centrifuge tube) that has passed through the filter.

Approximately 4 mL of the test sample was loaded into the filter unit and spun for a pre-determined time so that approximately 2 mL of filtrate was obtained. Both filtrate and retentate were collected and filtered through a 0.2m syringe filter prior to the PBMC-IL-6 assay. Each sample was separately tested using NMWL filters at 30 kDa, 50 kDa, and 100 kDa.

### Results

The samples tested using the above procedure included a positive control (Comparative Example A) and a negative control (Comparative Example B). Comparative Example A was a solution of 0.5 EU/mL lipopolysaccharide. Comparative Example B was RPMI with 2% plasma. Tests were also performed on peritoneal dialysis solution samples thatwere prepared from different batches of icodextrin based peritoneal dialysis solutions (Examples 1 and 2).

The samples used for Examples 1 and 2 were from batches of icodextrin-based peritoneal dialysis solutions that had previously been used in patients treated for peritoneal dialysis. The peritoneal dialysis solution in Example 1 had been found to cause aseptic peritonitis in some patients who used the peritoneal dialysis solution. The peritoneal dialysis solution prepared in Example 2 had not caused aseptic peritonitis in patients who used the peritoneal dialysis solution.

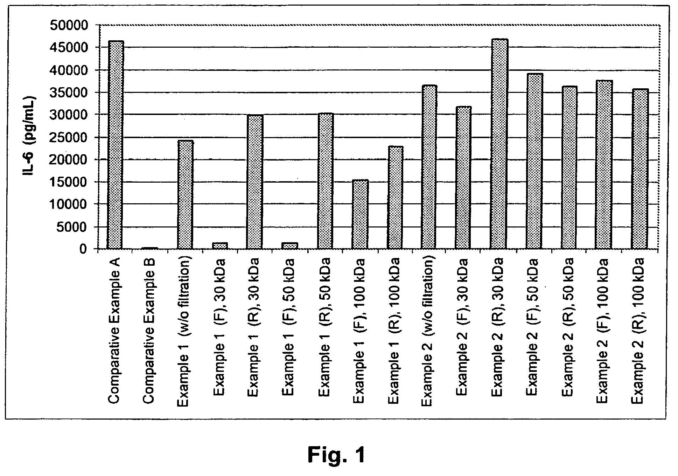

The IL-6 responses of samples of each solution were measured using the procedure described above. Additionally, the solutions of Examples 1 and 2 were subjected to filtration steps using the procedure previously described at molecular weight cutoffs of 30 kDa, 50 kDa, and 100 kDa. The filtrate and retentate were tested for IL-6 response at each cutoff level. The results are illustrated in Fig. 1. Each bar in Fig. 1 is labeled with the identity of the sample. The IL-6 results of the unfiltered samples are indicated by "w/o filtration." The IL-6 results for retentate samples are designated "R" and for filtrate samples designated "F." The last number of the label indicates the NMWL of the filter used in the test.

As illustrated in Fig. 1, it can be seen that, as expected, Comparative Example A exhibited a strong IL-6 response and that Comparative Example B exhibited virtually no IL-6 response. The unfiltered solutions of Examples 1 and 2 both exhibited high IL-6 responses (about 24,000 pg/mL and 36,000 pg/mL, respectively). Thus, the test results show that the solutions of Examples 1 and 2 were both contaminated with something that provoked an IL-6 response. Because only the icodextrin used in Example 1 had caused aseptic peritonitis, it is apparent that an IL-6 test alone was insufficient to determine if a solution would cause aseptic peritonitis.

Turning to the results for the retentate and the filtrate of Example 1, it can be seen that depending on the NMWL level, significant differences exist between the IL-6 response in the retentate and the filtrate. For the 30 kDa and 50 kDa samples, the IL-6 response of the retentate is several orders of magnitude higher than the IL-6 response of the filtrate. For the 100 kDa samples, the difference in IL-6 response is smaller. This difference in response based on molecular weight indicates that the contaminant that caused the aseptic peritonitis probably has a molecular weight between 30 kDa and 100 kDa.

In contrast, for Example 2, only a small difference is seen between the IL-6 response results for the filtrate and retentate samples, regardless of the NMWL level. These results indicate that the contaminant remains relatively equally distributed between the retentate and filtrate despite the filtration, and that the contaminant has a molecular weight less than 30 kDa.

Cytokine response tests were conducted for Comparative Example C (a positive control), Comparative Example D (a negative control) and additional dialysis solution samples that were prepared from different batches of icodextrin-based peritoneal dialysis solutions (Examples 3, 4, and 5). The samples used for Examples 3, 4, and 5 were from batches of icodextrin based peritoneal dialysis solutions that had previously been used in patients for peritoneal dialysis. The peritoneal dialysis solution in Examples 3 and 4 had been found to cause aseptic peritonitis in some patients who used the peritoneal dialysis solution. The peritoneal dialysis solution in Example 5 had not caused aseptic peritonitis in patients who used the peritoneal dialysis solution.

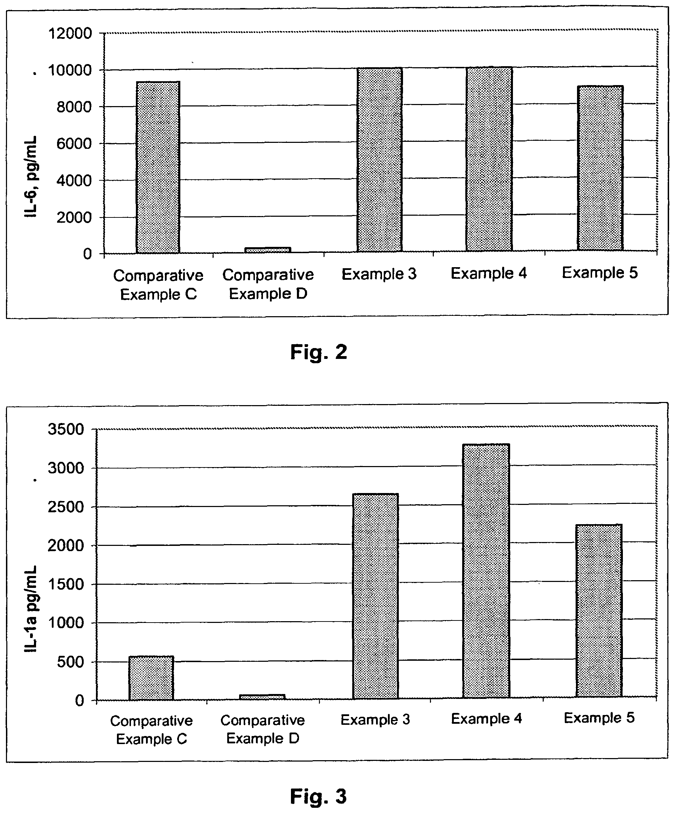

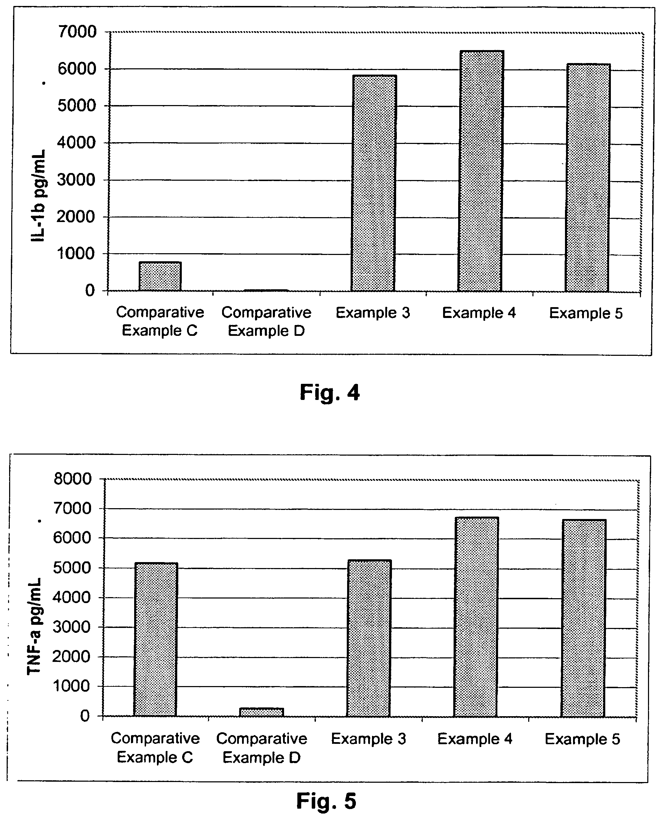

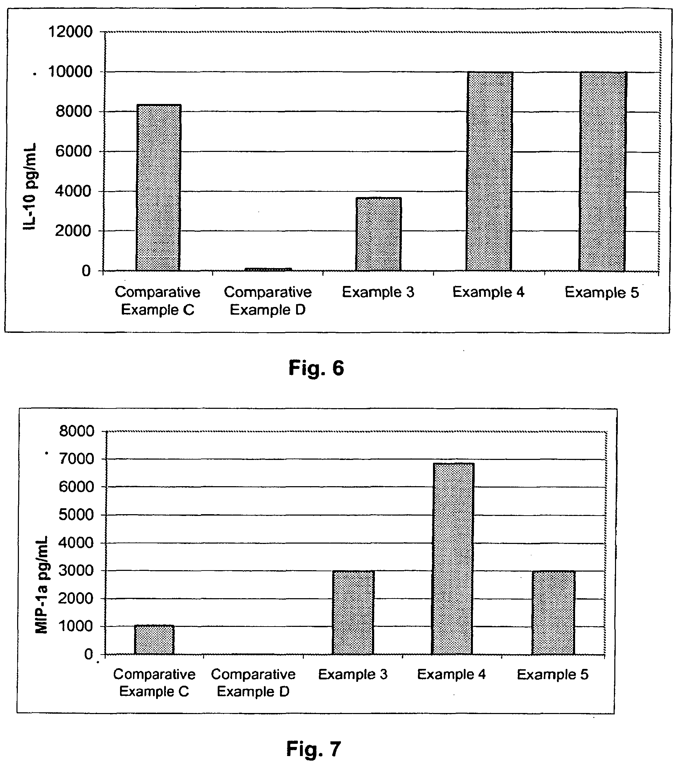

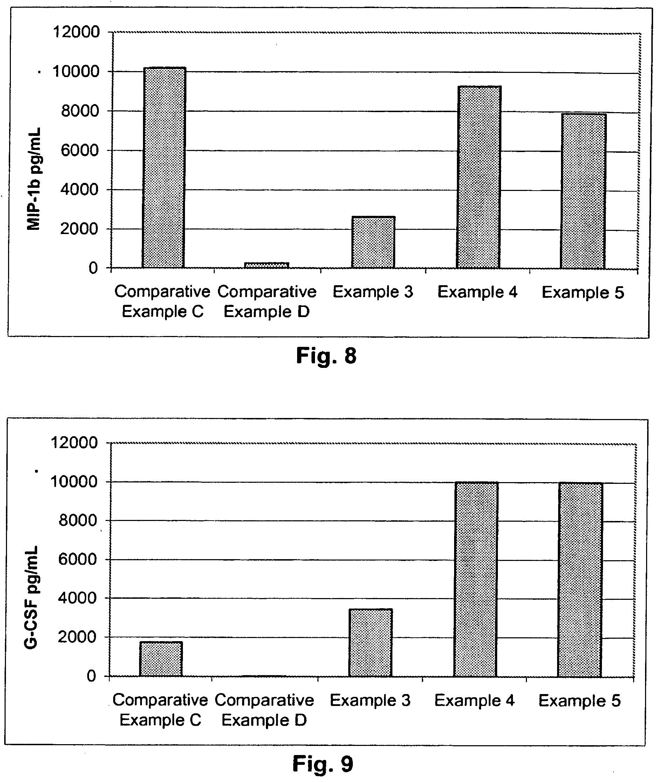

The cytokine responses are shown in Figs. 2-10 for IL-6, IL-1a, IL-1b, TNF-a, IL-10, MIP-Ia, MIP-1b, G-CSF, and GM-CSF, respectively. It can be seen that in each case, Comparative Example C and Examples 3, 4, and 5 each exhibited a significant cytokine response, and Comparative Example D (the negative control) exhibited a very low response.

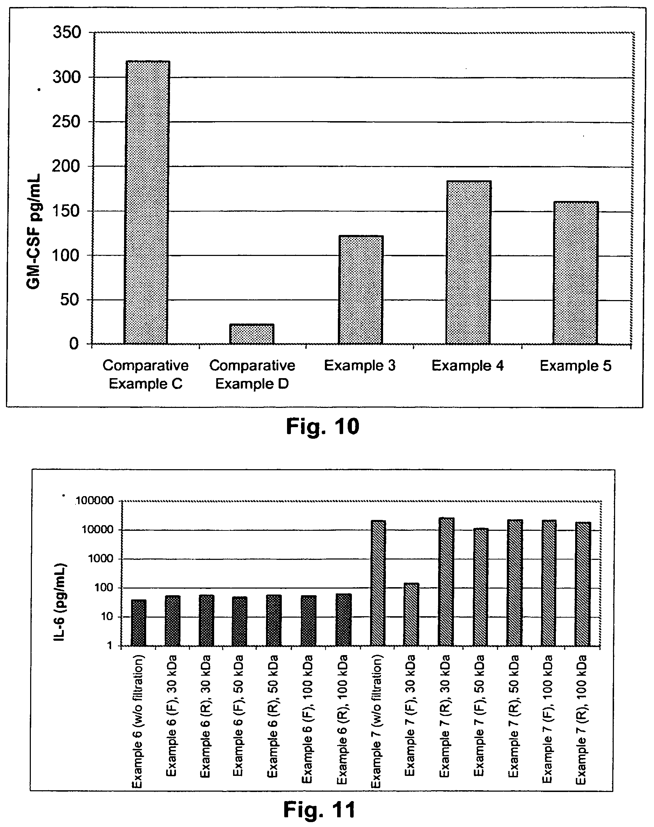

Examples 6 and 7 were dialysis solution samples from different batches of icodextrin-based peritoneal dialysis solutions. The solutions of Examples 6 and 7 were subjected to filtration steps using the procedure previously described at molecular weight cutoffs of 30 kDa, 50 kDa, and 100 kDa. The filtrate and retentate were tested for IL-6 response at each cutoff level. Fig. 11 illustrates the IL-6 response on a logarithmic scale for Examples 6 and 7.

For Example 6, the IL-6 response was very small for both the filtrate and retentate samples, regardless of the NMWL level. These results indicate that the contaminant remained relatively equally distributed between the retentate and filtrate despite the filtration. Thus, the sample of Example 6 was suitable for use.

For Example 7, it can be seen that for the sample filtered at 30 kDa, the IL-6 response of the retentate was much larger than the IL-6 response of the filtrate. This indicates that a contaminate with a molecular weight greater than 30 kDa was present in the solution of Example 7. Thus, the dialysis fluid of Example 7 was not suitable for use.

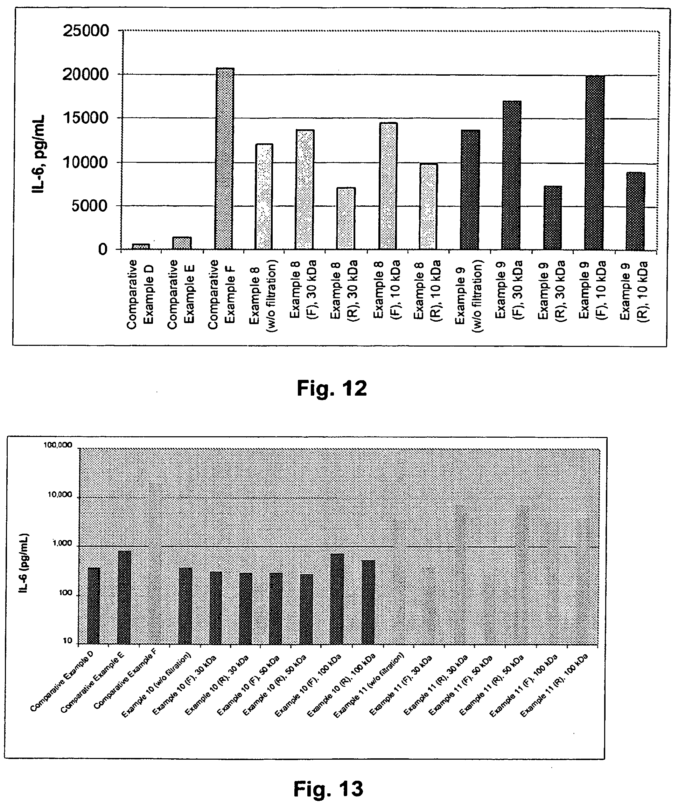

Fig. 12 illustrates the IL-6 response for Example 8, Example 9, Comparative Example D, Comparative Example E, and Comparative Example F. Examples 8 and 9 were fluid samples prepared from different samples of icodextrin. Comparative Example D was a negative control of RPMI, Comparative Example E was a negative control of an uncontaminated peritoneal dialysis fluid, and Comparative Example F was a positive control of contaminated peritoneal dialysis fluid. The samples were filtered and tested at 10 kDa and 30 kDa. It can be seen that for both Examples 8 and 9, the IL-6 response of the filtrate was greater than the IL-6 response of the retentate, at both filtration levels. This indicates that the contaminants in the icodextrin passed through the filters and thus have a molecular weight less than 10 kDa. Thus, the icodextrin was suitable for use.

Fig. 13 illustrates the IL-6 response on a logarithmic scale for Comparative Example D, Comparative Example E, Comparative Example F, and Examples 10 and 11. Examples 10 and 11 werefluid samples prepared from different samples of icodextrin. The samples were filtered and tested at 30 kDa, 50 kDa, and 100 kDa. For Example 10, the IL-6 response was relatively low for each fraction (comparable to the responses of the negative controls, Comparative Examples D and E), and each fraction showed a similar response. Thus, the icodextrin was suitable for use. For Example 11, the IL-6 response of the retentate was greater than the IL-6 response of the filtrate, at 30 kDa and 50 kDa. Because the contaminant had a size greater than 30 kDa, the icodextrin was not suitable for use.

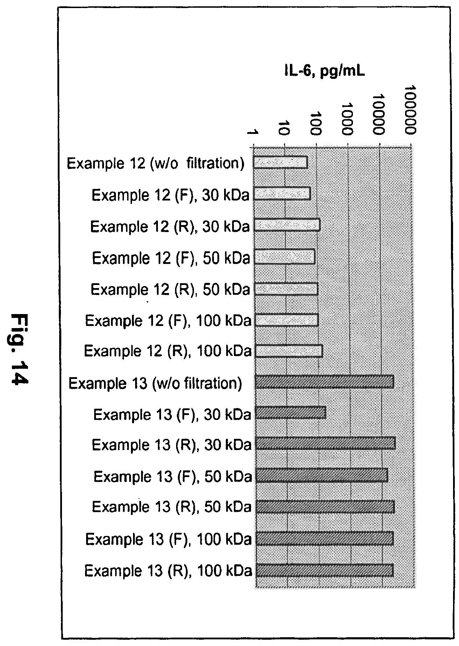

Fig. 14 illustrates the IL-6 response on a logarithmic scale for Examples 12 and 13. Examples 12 and 13 were samples of icodextrin-based peritoneal dialysis solution. The samples were filtered and tested at 30 kDa, 50 kDa, and 100 kDa. For Example 12, the IL-6 response was relatively low for each fraction and each fraction showed a similar response. Thus, the peritoneal dialysis fluid of Example 12 was suitable for use. For Example 13, the IL-6 response of the retentate was greater than the IL-6 response of the filtrate at 30 kDa. Because the contaminant had a size greater than 30 kDa, the icodextrin was not suitable for use.

Based on these test results, it can be seen that using a cytokine response test in combination with a filtering step provides an improved method to test for the presence of contaminants in a dialysis solution.

It should be understood that various changes and modifications to the presently preferred embodiments described herein will be apparent to those skilled in the art. It is therefore intended that such changes and modifications be covered by the appended claims.

## Claims

1. An in vitro method of testing a glucose polymer or a glucose polymer derivative to determine the presence of peptidogylcan, comprising:
providing a sample of the glucose polymer or glucose polymer derivative;
filtering the sample at a molecular weight cutoff of 10 kDa to 100 kDa to obtain a retentate portion and a filtrate portion;
adding the retentate portion to a first assay;
adding the filtrate portion to a second assay;
adding a reagent to each of the first assay and the second assay, wherein the reagent produces a proinflammatory response;
measuring the proinflammatory response of each of the first assay and the second assay;
comparing the proinflammatory response of the first assay to the proinflammatory response of the second assay and determining if the proinflammatory response of the second assay, as expressed in pg/ML, is greater than, less than, or equal to 50% of the proinflammatory response of the first assay, as expressed in pg/mL, to determine if the glucose polymer or glucose polymer derivative is suitable for use;
wherein if the proinflammatory response of the second assay, as expressed in pg/mL, is greater than or equal to 50% of the proinflammatory response of the first assay, as expressed in pg/mL, further comprising the step of accepting the glucose polymer or glucose polymer derivative for use, and wherein if the proinflammatory response of the second essay, as expressed in pg/mL, is less than 50% of the proinflammatory response of the first assay, as expressed in pg/mL, further comprising the step of rejecting the glucose polymer or glucose polymer derivative for use;
wherein the reagent comprises peripheral blood mononuclear cells or monocytic cell line cells; and
wherein the proinflammatory response is an interleukin-6 response.

2. An in vitro method of testing a glucose polymer or a glucose polymer derivative to determine the presence of peptidogylcan, comprising:
providing a first sample of the glucose polymer or glucose polymer derivative;
adding a reagent to the first sample, wherein the reagent produces an interleukin-6 response;
measuring an interleukin-6 response induced by the first sample;
providing a second sample of the glucose polymer or glucose polymer derivative;
filtering the second sample at a molecular weight cutoff of greater than or equal to about 30 kDa and less than or equal to about 100 kDa to obtain a retentate portion and a filtrate portion;
adding the reagent to each of the retentate portion and the filtrate portion;
measuring an interleukin-6 response induced by each of the retentate portion and the filtrate portion;
comparing the interleukin-6 response induced by the retentate portion to the interleukin-6 response induced by the filtrate portion and determining if the proinflammatory response of the filtrate portion, as expressed in pg/mL, is greater than, less than, or equal to 50% of the proinflammatory response of the retentate portion, as expressed in pg/mL, to determine if the glucose polymer or glucose polymer derivative is suitable for use;
wherein if the proinflammatory response induced by the filtrate portion, as expressed in pg/mL, is greater than or equal to 50% of the proinflammatory response induced by the retentate portion, as expressed in pg/mL, further comprising the step of accepting the glucose polymer or the glucose polymer derivative for use, and wherein if the proinflammatory response induced by the filtrate portion, as expressed in pg/mL, is less than 50% of the proinflammatory response induced by the retentate portion, as expressed in pg/mL, further comprising the step of rejecting the glucose polymer or the glucose polymer derivative for use;
wherein the reagent comprises peripheral blood mononuclear cells or monocytic cell line cells.

3. An in vitro method of testing a peritoneal dialysis solution to determine the presence of peptidogylcan, comprising:
providing a sample of the peritoneal dialysis solution, wherein the peritoneal dialysis solution comprises a glucose polymer or a glucose polymer derivative;
filtering the sample at a molecular weight cutoff of 10 kDa to 100 kDa to obtain a retentate portion and a filtrate portion;
adding the retentate portion to a first assay;
adding the filtrate portion to a second assay;
adding a reagent to each of the first assay and the second assay, wherein the reagent produces a proinflammatory response;
measuring the proinflammatory response of each of the first assay and the second assay;
comparing the proinflammatory response of the first assay to the proinflammatory response of the second assay and determining if the proinflammatory response of the second assay, as expressed in pg/mL, is greater than, less than, or equal to 50% of the proinflammatory response of the first assay, as expressed in pg/mL, to determine if the peritoneal dialysis solution is suitable for use;
wherein if the proinflammatory response of the second assay, as expressed in pg/mL, is greater than or equal to 50% of the proinflammatory response of the first assay, as expressed in pg/mL, further comprising the step of accepting the peritoneal dialysis solution for use, and wherein if the proinflammatory response of the second essay, as expressed in pg/mL, is less than 50% of the proinflammatory response of the first assay, as expressed in pg/mL, further comprising the step of rejecting the peritoneal dialysis solution for use;
wherein the reagent comprises peripheral blood mononuclear cells or monocytic cell line cells; and
wherein the proinflammatory response is an interleukin-6 response.

4. The method of Claim 1 or 4, wherein the specified molecular weight cutoff is greater than or equal to about 30 kDa and less than or equal to about 100 kDa.

5. The method of any one of claims 1, 2 and 3, wherein the specified molecular weight cutoff is greater than or equal to about 50 kDa and less than or equal to about 100 kDa.

6. The method of Claim 1 or 3, wherein the glucose polymer or glucose polymer derivative is icodextrin.

## Patentansprüche

1. *In vitro* Verfahren zum Testen eines Glucosepolymers oder eines Glucosepolymer-Derivats, um das Vorhandensein von Peptidoglycan festzustellen, umfassend:
Bereitstellen einer Probe des Glucosepolymers oder Glucosepolymer-Derivats,
Filtern der Probe bei einer Molekulargewichtsgrenze von 10 kDa bis 100 kDa, um einen Retentatanteil und einen Filtratanteil zu erhalten,
Zugeben des Retentatanteils zu einem ersten Assay,
Zugeben des Filtratanteils zu einem zweiten Assay,
Zugeben eines Reagens zu jeweils dem ersten Assay und dem zweiten Assay, wobei das Reagens eine proinflammatorische Antwort hervorruft,
Messen der proinflammatorischen Antwort von jeweils dem ersten Assay und dem zweiten Assay,
Vergleichen der proinflammatorischen Antwort des ersten Assays mit der proinflammatorischen Antwort des zweiten Assays und Bestimmen, ob die proinflammatorische Antwort des zweiten Assays, ausgedrückt in pg/mL, größer, kleiner oder gleich ist zu 50% der proinflammatorischen Antwort des ersten Assays, ausgedrückt in pg/mL, um zu bestimmen, ob das Glucosepolymer oder Glucosepolymer-Derivat zur Verwendung geeignet ist,
wobei, wenn die proinflammatorische Antwort des zweiten Assays, ausgedrückt in pg/mL, größer oder gleich ist zu 50% der proinflammatorischen Antwort des ersten Assays, ausgedrückt in pg/mL, weiter der Schritt des Annehmens des Glucosepolymers oder Glucosepolymer-Derivats zur Verwendung umfaßt ist, und wobei, wenn die proinflammatorische Antwort des zweiten Assays, ausgedrückt in pg/mL, kleiner ist als 50% der proinflammatorischen Antwort des ersten Assays, ausgedrückt in pg/mL, weiter der Schritt des Ablehnens des Glucosepolymers oder Glucosepolymer-Derivats zur Verwendung umfaßt ist,
wobei das Reagens mononukleäre Zellen des peripheren Bluts oder Zellen einer monocytischen Zelllinie umfaßt, und
wobei die proinflammatorische Antwort eine Interleukin-6-Antwort ist.

2. *In vitro* Verfahren zum Testen eines Glucosepolymers oder Glucosepolymer-Derivats, um das Vorhandensein von Peptidoglycan festzustellen, umfassend:
Bereitstellen einer ersten Probe des Glucosepolymers oder Glucosepolymer-Derivats,
Zugeben eines Reagens zu der ersten Probe, wobei das Reagens eine Interleukin-6-Antwort hervorruft,
Messen einer Interleukin-6-Antwort, die von der ersten Probe induziert wurde,
Bereitstellen einer zweiten Probe des Glucosepolymers oder Glucosepolymer-Derivats,
Filtern der zweiten Probe bei einer Molekulargewichtsgrenze von größer oder gleich etwa 30 kDa und kleiner oder gleich etwa 100 kDa, um einen Retentat-anteil und einen Filtratanteil zu erhalten,
Zugeben des Reagens zu jeweils dem Retentatanteil und dem Filtratanteil,
Messen einer Interleukin-6-Antwort, die jeweils von dem Retentatanteil und dem Filtratanteil induziert wurde,
Vergleichen der Interleukin-6-Antwort, die von dem Retentatanteil induziert wurde, mit der Interleukin-6-Antwort, die von dem Filtratanteil induziert wurde, und Bestimmen, ob die proinflammatorische Antwort des Filtratanteils, ausgedrückt in pg/mL, größer, kleiner oder gleich ist zu 50% der proinflammatorischen Antwort des Retentatanteils, ausgedrückt in pg/mL, um zu bestimmen, ob das Glucosepolymer oder Glucosepolymer-Derivat zur Verwendung geeignet ist,
wobei, wenn die proinflammatorische Antwort, die durch den Filtratanteil induziert wurde, ausgedrückt in pg/mL, größer oder gleich ist zu 50% der proinflammatorischen Antwort, die von dem Retentatanteil induziert wurde, ausgedrückt in pg/mL, weiter der Schritt des Annehmens des Glucosepolymers oder Glucosepolymer-Derivats zur Verwendung umfaßt ist, und wobei, wenn die proinflammatorische Antwort, die durch den Filtratanteil induziert wurde, ausgedrückt in pg/mL, kleiner ist als 50% der proinflammatorischen Antwort, die von dem Retentatanteil induziert wurde, ausgedrückt in pg/mL, weiter der Schritt des Ablehnens der Glucosepolymers oder Glucosepolymer-Derivats zur Verwendung umfaßt ist,
wobei das Reagens mononukleäre Zellen des peripheren Bluts oder Zellen einer monocytischen Zelllinie umfaßt.

3. *In vitro* Verfahren zum Testen einer peritonealen Dialyselösung, um das Vorhandensein von Peptidoglycan festzustellen, umfassend:
Bereitstellen einer Probe der peritonealen Dialyselösung, wobei die peritoneale Dialyselösung ein Glucosepolymer oder ein Glucosepolymer-Derivat umfaßt,
Filtern der Probe bei einer Molekulargewichtsgrenze von 10 kDa bis 100 kDa, um einen Retentatanteil und einen Filtratanteil zu erhalten,
Zugeben des Retentatanteils zu einem ersten Assay,
Zugeben des Filtratanteils zu einem zweiten Assay,
Zugeben eines Reagens zu jeweils dem ersten Assay und dem zweiten Assay, wobei das Reagens eine proinflammatorische Antwort hervorruft,
Messen der proinflammatorischen Antwort von jeweils dem ersten Assay und dem zweiten Assay,
Vergleichen der proinflammatorischen Antwort des ersten Assays mit der proinflammatorischen Antwort des zweiten Assays und Bestimmen, ob die proinflammatorische Antwort des zweiten Assays, ausgedrückt in pg/mL, größer, kleiner oder gleich ist zu 50% der proinflammatorischen Antwort des ersten Assays, ausgedrückt in pg/mL, um zu bestimmen, ob die peritoneale Dialyselösung zur Verwendung geeignet ist,
wobei, wenn die proinflammatorische Antwort des zweiten Assays, ausgedrückt in pg/mL, größer oder gleich ist zu 50% der proinflammatorischen Antwort des ersten Assays, ausgedrückt in pg/mL, weiter der Schritt des Annehmens der peritonealen Dialyselösung zur Verwendung umfaßt ist, und wobei, wenn die proinflammatorische Antwort des zweiten Assays, ausgedrückt in pg/mL, kleiner ist als 50% der proinflammatorischen Antwort des ersten Assays, ausgedrückt in pg/mL, weiter der Schritt des Ablehnens der peritonealen Dialyselösung zur Verwendung umfaßt ist,
wobei das Reagens mononukleäre Zellen des peripheren Bluts oder Zellen einer monocytischen Zelllinie umfaßt, und
wobei die proinflammatorische Antwort eine Interleukin-6-Antwort ist.

4. Verfahren nach Anspruch 1 oder 3, wobei die bestimmte Molekulargewichtsgrenze größer oder gleich ist zu etwa 30 kDa und kleiner oder gleich ist zu etwa 100 kDa.

5. Verfahren nach einem der Ansprüche 1, 2 und 3, wobei die bestimmte Molekulargewichtsgrenze größer oder gleich ist zu etwa 50 kDa und kleiner oder gleich ist zu etwa 100 kDa.

6. Verfahren nach Anspruch 1 oder 3, wobei das Glucosepolymer oder Glucosepolymer-Derivat Icodextrin ist.

## Revendications

1. Procédé d'essai *in vitro* d'un polymère de glucose ou d'un dérivé de polymère de glucose, pour déterminer la présence d'un peptidoglycane, comprenant les étapes consistant à :
fournir un échantillon du polymère de glucose ou du dérivé de polymère de glucose ;
filtrer l'échantillon à un seuil de coupure de poids moléculaire de 10 kDa à 100 kDa pour obtenir une portion de rétentat et une portion de filtrat ;
ajouter la portion de rétentat à un premier dosage ;
ajouter la portion de filtrat à un second dosage ;
ajouter un réactif à chacun du premier dosage et du second dosage, dans lequel le réactif produit une réponse pro-inflammatoire ;
mesurer la réponse pro-inflammatoire de chacun du premier dosage et du second dosage ;
comparer la réponse pro-inflammatoire du premier dosage à la réponse pro-inflammatoire du second dosage et déterminer si la réponse pro-inflammatoire du second dosage, telle qu'exprimée en pg/mL, est supérieure, inférieure ou égale à 50 % de la réponse pro-inflammatoire du premier dosage, telle qu'exprimée en pg/mL, afin de déterminer si le polymère de glucose ou le dérivé de polymère de glucose convient à l'utilisation ;
dans lequel, si la réponse pro-inflammatoire du second dosage, telle qu'exprimée en pg/mL, est supérieure ou égale à 50 % de la réponse pro-inflammatoire du premier dosage, telle qu'exprimée en pg/mL, comprenant en outre l'étape consistant à accepter le polymère de glucose ou le dérivé de polymère de glucose pour l'utilisation, et dans lequel, si la réponse pro-inflammatoire du second dosage, telle qu'exprimée en pg/mL, est inférieure à 50 % de la réponse pro-inflammatoire du premier dosage, telle qu'exprimée en pg/mL, comprenant en outre l'étape consistant à rejeter le polymère de glucose ou le dérivé de polymère de glucose pour l'utilisation ;
dans lequel le réactif comprend des cellules mononuclées de sang périphérique ou des cellules de lignée cellulaire monocytique ; et
dans lequel la réponse pro-inflammatoire est une réponse d'interleukine-6.

2. Procédé d'essai *in vitro* d'un polymère de glucose ou d'un dérivé de polymère de glucose, pour déterminer la présence d'un peptidoglycane, comprenant les étapes consistant à :
fournir un premier échantillon du polymère de glucose ou du dérivé de polymère de glucose ;
ajouter un réactif au premier échantillon, dans lequel le réactif produit une réponse de l'interleukine-6 ;
mesurer une réponse de l'interleukine-6 induite par le premier échantillon ;
fournir un second échantillon du polymère de glucose ou du dérivé de polymère de glucose ;
filtrer le second échantillon à un seuil de coupure de poids moléculaire supérieur ou égal à environ 30 kDa ou inférieur ou égal à environ 100 kDa afin d'obtenir une portion de rétentat et une portion de filtrat ;
ajouter le réactif à chacune de la portion de rétentat et de la portion de filtrat ;
mesurer une réponse de l'interleukine-6 induite par chacune de la portion de rétentat et de la portion de filtrat ;
comparer la réponse de l'interleukine-6 induite par la portion de rétentat à la réponse de l'interleukine-6 induite par la portion de filtrat et déterminer si la réponse pro-inflammatoire de la portion de filtrat, telle qu'exprimée en pg/mL, est supérieure, inférieure, ou égale à 50 % de la réponse pro-inflammatoire de la portion de rétentat, telle qu'exprimée en pg/mL, afin de déterminer si le polymère de glucose ou le dérivé de polymère de glucose convient à l'utilisation ;
dans lequel, si la réponse pro-inflammatoire induite par la portion de filtrat, telle qu'exprimée en pg/mL, est supérieure ou égale à 50 % de la réponse pro-inflammatoire induite par la portion de rétentat, telle qu'exprimée en pg/mL, comprenant en outre l'étape consistant à accepter le polymère de glucose ou le dérivé de polymère de glucose pour l'utilisation, et dans lequel, si la réponse pro-inflammatoire induite par la portion de filtrat, telle qu'exprimée en pg/mL, est inférieure à 50 % de la réponse pro-inflammatoire induite par la portion de rétentat, telle qu'exprimée en pg/mL, comprenant en outre l'étape consistant à rejeter le polymère de glucose ou le dérivé de polymère de glucose pour l'utilisation ;
dans lequel le réactif comprend des cellules mononuclées de sang périphérique ou des cellules de lignée cellulaire monocytique.

3. Procédé d'essai *in vitro* d'une solution de dialyse péritonéale pour déterminer la présence d'un peptidoglycane, comprenant les étapes consistant à :
fournir un échantillon de la solution de dialyse péritonéale, dans lequel la solution de dialyse péritonéale comprend un polymère de glucose ou un dérivé de polymère de glucose ;
filtrer l'échantillon à un seuil de coupure de poids moléculaire de 10 kDa à 100 kDa afin d'obtenir une portion de rétentat et une portion de filtrat ;
ajouter la portion de rétentat à un premier dosage ;
ajouter la portion de filtrat à un second dosage ;
ajouter un réactif à chacun du premier dosage et du second dosage, dans lequel le réactif produit une réponse pro-inflammatoire ;
mesurer la réponse pro-inflammatoire de chacun du premier dosage et du second dosage ;
comparer la réponse pro-inflammatoire du premier dosage à la réponse pro-inflammatoire du second dosage et déterminer si la réponse pro-inflammatoire du second dosage, telle qu'exprimée en pg/mL, est supérieure, inférieure, ou égale à 50 % de la réponse pro-inflammatoire du premier dosage, telle qu'exprimée en pg/mL, afin de déterminer si la solution de dialyse péritonéale convient à l'utilisation ;
dans lequel si la réponse pro-inflammatoire du second dosage, telle qu'exprimée en pg/mL, est supérieure ou égale à 50 % de la réponse pro-inflammatoire du premier dosage, telle qu'exprimée en pg/mL, comprenant en outre l'étape consistant à accepter la solution de dialyse péritonéale pour l'utilisation, et dans lequel si la réponse pro-inflammatoire du second dosage, telle qu'exprimée en pg/mL, est inférieure à 50 % de la réponse pro-inflammatoire du premier dosage, telle qu'exprimée en pg/mL, comprenant en outre l'étape consistant à rejeter la solution de dialyse péritonéale pour l'utilisation ;
dans lequel le réactif comprend des cellules mononuclées de sang périphérique ou des cellules de lignée cellulaire monocytique ; et
dans lequel la réponse pro-inflammatoire est une réponse de l'interleukine-6.

4. Procédé selon la revendication 1 ou 3, dans lequel le seuil de coupure de poids moléculaire spécifié est supérieur ou égal à environ 30 kDa ou inférieur ou égal à environ 100 kDa.

5. Procédé selon l'une quelconque des revendications 1, 2 et 3, dans lequel le seuil de coupure de poids moléculaire spécifié est supérieur ou égal à environ 50 kDa ou inférieur ou égal à environ 100 kDa.

6. Procédé selon la revendication 1 ou 3, dans lequel le polymère de glucose ou le dérivé de polymère de glucose est l'icodextrine.
